# EUROPÄISCHE PATENTSCHRIFT

(11) **EP 0 588 384 B1**
(45) Veröffentlichungstag und Bekanntmachung des Hinweises auf die Patenterteilung: **30.07.1997**
(21) Anmeldenummer: 93119161.3
(22) Anmeldetag: 24.08.1990
(51) Int. Cl.: A61F 9/02

(54) **Schutzbrille gegen augenschädliche Strahlung**
Safety glasses against eye-damaging rays
Lunette de protection contre les rayons nocifs pour les yeux

(30) Priorität: 26.08.1989 DE 8910235 U
(43) Veröffentlichungstag der Anmeldung: 23.03.1994
(62) Teilanmeldung aus: 90116221.4
(73) Patentinhaber: Carl Zeiss, D-89520 Heidenheim (Brenz) (DE); CARL ZEISS-STIFTUNG HANDELND ALS CARL ZEISS, D-89518 Heidenheim (Brenz) (DE)
(72) Erfinder: Paysan, Heinz-Wilhelm, D-73432 Waldhausen (DE); Gaiser, Hans, D-72768 Reutlingen (DE); Grimm, Wolfgang, Dr., D-89520 Heidenheim (DE); Gutbrod, Heinz, D-71229 Leonberg (DE); Schürle, Hermann, D-73430 Aalen (DE)

(56) Entgegenhaltungen:
- AT-B- 388 659
- DE-A- 3 111 577
- DE-A- 3 503 393

## Beschreibung

Die vorliegende Erfindung betrifft eine Schutzbrille gegen augenschädliche Strahlung, bestehend aus Schutzfiltern und einer zu deren Aufnahme dienenden Brillenfassung.

Solche Schutzbrillen müssen beim Arbeiten z.B. mit Lasern ab der Gefahrenklasse 3b getragen werden, um die Augen vor schädlicher Strahlung zu schützen. Dabei ist es wesentlich, daß die Schutzbrille die Augen des Benutzers nicht nur zuverlässig vor Strahlung schützt, die im wesentlichen von vorne kommt, sondern daß auch aus anderen Richtungen auftreffende Strahlung von den Augen abgehalten wird.

Dazu dienen Fassungen wie sie beispielsweise aus der DE-AS 20 62 829 bekannt sind. Diese Fassungen tragen im Schläfenbereich jeweils eine mit dem Ohrbügel verbundene Seitenblende, die im geöffneten Zustand am zugeordneten Augenrand der Fassung anliegt. Derartige Fassungen schützen nicht die Bereiche ober- und unterhalb des Augenrandes und es besteht die Gefahr, daß die Seitenblende nicht satt am zugeordneten Augenrand der Fassung anliegt und somit im kritischen Bereich ein Schlitz entsteht.

Aus dem DE-GM 85 32 493 ist eine Laserschutzbrille bekannt, bei der neben schläfenseitigen Seitenblenden auch im oberen Fassungsbereich Blenden vorgesehen sind, die den Bereich bis zur Stirn des Brillenträgers abdecken. Auch hier besteht die Gefahr, daß sich ein Schlitz zwischen Seitenblende und Augenrand der Fassung bildet; zudem sind Bereiche am unteren Rand der Fassung nicht abgeschirmt.

Diese bekannten Fassungen bauen auf den üblichen sogenannten Arbeitsschutzbrillen auf, an deren Fassung die notwendigen Blenden angesetzt werden. Dadurch werden diese Fassungen relativ teuer.

Aus der US-PS 4 527 291 ist eine Sicherheitsbrille bekannt, bei welcher die Ohrbügel von außen an der Brillenfassung befestigt sind. Diese Art der Ohrbügelbefestigung führt zu Kanten, welche ein ungewolltes Abreißen der Sicherheitsbrille ermöglichen. Außerdem besteht zwischen Brillenfassung und Brillenträgergesicht große, nicht abgedeckte Bereiche.

In letzter Zeit ist auf dem Markt die Laserschutzbrille L-04 der Firma Laser-Vision GmbH erhältlich, bei der sich die Fassung temporal bis in den Schläfenbereich erstreckt und erst dort Scharniere zum schwenkbaren Befestigen der Ohrbügel trägt. Bei dieser Fassung sind durchsichtige, mit Schutzfiltern versehene Seitenblenden und Blenden am oberen Fassungsrand vorgesehen. Da alle Blenden an einer Grundfassung gesondert befestigt sind, ist auch diese Fassung relativ teuer.

Bekannt sind auch sogenannte Korbfassungen für Laserschutzbrillen. Diese Fassungen ähneln in ihrem Aufbau den Taucherschutzbrillen, z.B. DE-PS 36 16 253. Der die Augenfassungen umgebende Fassungsteil besteht aus weichelastischem Material und deckt den Bereich zwischen den Schutzfiltern und dem Gesicht des Benutzers voll ab. Solche Korbfassungen werden meist für Besucher oder für Brillenträger verwendet, da sie über der normalen Korrekturfassung getragen werden können. Die erwähnten Korbfassungen weisen keine Ohrbügel auf; sie werden mit einem elastischen Band am Kopf des Trägers gehalten.

DE-A-3 111 577 offenbart eine Schutzbrille mit den Merkmalen des Oberbegriffs von Anspruch 1.

Die Aufgabe der vorliegenden Erfindung ist es eine Schutzbrille so auszubilden, daß sie eine gute Hinterlüftung bietet, ohne daß schädliche Strahlung in die Augen eines Brillenträgers dringen kann.

Diese Aufgabe wird erfindungsgemäß durch eine Schutzbrille gelöst, die nach den kennzeichnenden Merkmalen des Anspruchs 1 ausgebildet ist.

Die Erfindung wird im folgenden anhand der Ausführungsbeispiele darstellenden Figuren 1 - 11 der beigefügten Zeichnungen näher erläutert. Dabei zeigen:
- Fig. 1: eine perspektivische Darstellung einer Laserschutzbrille;
- Fig. 2: die Ansicht der Laserschutzbrille nach Fig. 1 von oben;
- Fig. 3: die Ansicht der Laserschutzbrille nach Fig. 1 von unten;
- Fig. 4: einen Schnitt entlang der Linie IV-IV der Fig. 1;
- Fig. 5: eine Teilansicht der Laserschutzbrille von oben mit teilweise eingeklapptem Ohrbügel;
- Fig. 6: eine perspektivische Darstellung einer Laserschutzbrille mit Lüftungsschlitzen;
- Fig. 7: einen Ohrbügel mit Lüftungsschlitzen im Schnitt;
- Fig. 8: einen zweiten Ohrbügel mit Lüftungsschlitzen im Schnitt;
- Fig. 9: einen Ohrbügel in seitlicher Ansicht;
- Fig. 10: einen Scharnierbereich im Schnitt von oben gesehen; und
- Fig. 11: einen Scharnierbereich im Schnitt von oben gesehen.

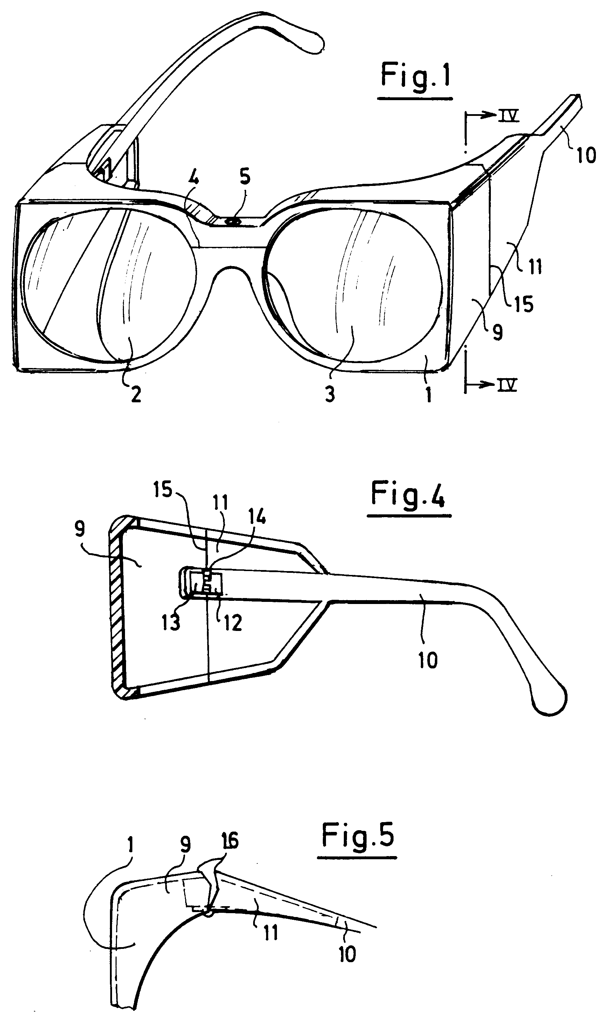

In Figur 1 ist das erste Fassungsteil der Laserschutzbrille mit (1) bezeichnet. Dieses Fassungsteil ist einstückig ausgebildet und trägt die Schutzfilter (2 und 3). Zum Einsetzen der Schutzfilter (2, 3) kann das Fassungsteil (1) bei (4) geschlitzt sein. Eine Schraube (5) dient nach dem Einsetzen der Schutzfilter (2, 3) zum strahlungsdichten Schließen des Schlitzes (4).

Wie insbesondere aus den Fig. 1, 2 und 3 ersichtlich ist ist das Fassungsteil (1) beidseitig temporal bis in den Schläfenbereich des Brillenträgers hineingezogen und bildet dort Seitenblenden, die auch als Schläfenplatten bezeichnet werden können.

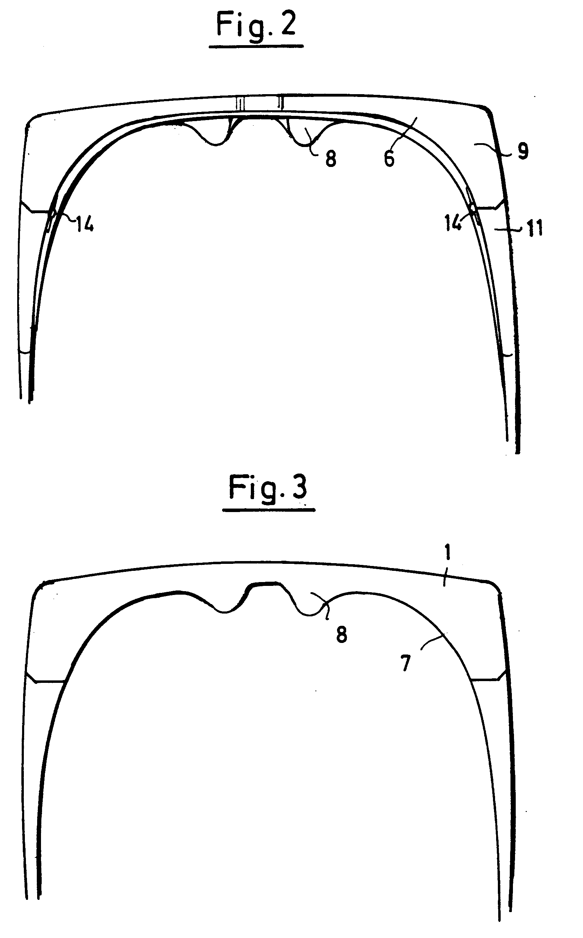

Wie Figur 2 zeigt ist mit dem Fassungsteil (1) eine obere Schutzblende (6) integriert, welche den Bereich zwischen der Fassung und der Stirn des Brillenträgers abdeckt.

Aus Figur 3 ist ersichtlich, daß der untere Teil des Fassungsteils (1) als Schutzblende (7) ausgebildet ist, welche den Bereich zwischen der Fassung und dem Gesicht des Brillenträgers überdeckt. Die untere Blende (7) geht direkt in die Nasenauflagen (8) über. Auch diese sind in das Fassungsteil (1) integriert.

Wie aus diesen Figuren ersichtlich ist, bildet das Fassungsteil (1) im Querschnitt eine zum Gesicht des Brillenträgers hin offene Schale, in deren Frontfläche die Schutzfilter eingesetzt sind.

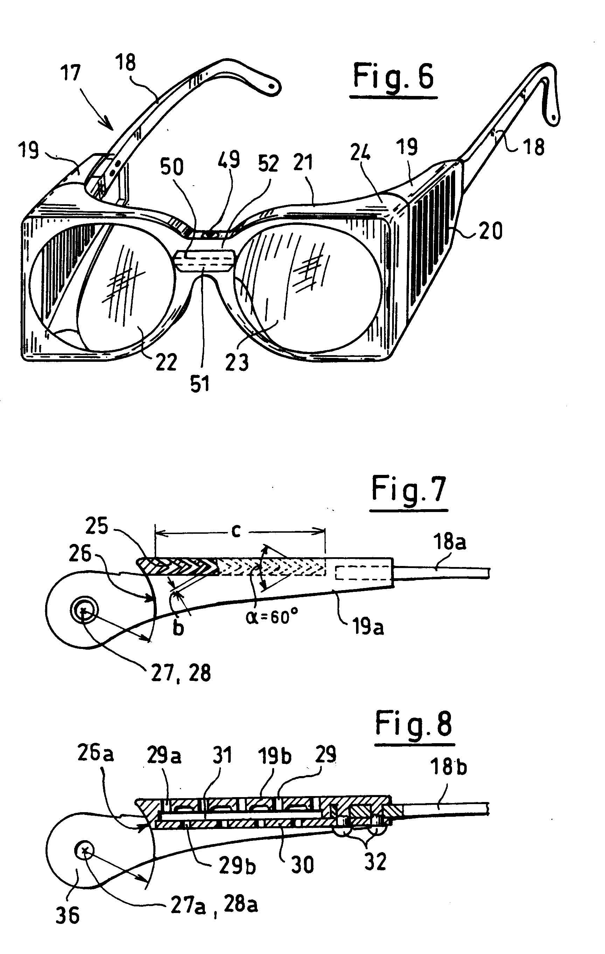

An jeder Schläfenplatte (9) ist ein Ohrbügel (10) drehbar befestigt. Wie Fig. 4 zeigt ist der Ohrbügel (10) in eine Platte (11) integriert, welche die Fortsetzung der Schläfenplatte (9) bildet. Mit der Schläfenplatte (9) ist ein erster Scharnierteil (12) fest verbunden, während der zweite Scharnierteil (13) mit der Platte (11) fest verbunden ist. Der Scharnierbolzen (14) bildet den Drehpunkt des Scharniers. Er ist auf der Innenseite der Fassung (1) angeordnet und zwar in der Ebene der Trennfuge (15) zwischen den beiden Platten (9 und 11).

Wie Figur 5 zeigt ist die Trennfuge so ausgebildet, daß sie abgeschrägte Teile (16) enthält, welche jeweils über die gesamte Wandstärke der beiden Platten (9 und 11) reichen. Diese beiden Abschrägungen (16) liegen bei geöffnetem Ohrbügel aufeinander. Durch diese Ausbildung der Trennfuge ist erreicht, daß auch bei nicht ganz geöffnetem Ohrbügel kein Schlitz entsteht, welcher für Strahlung durchlässig ist.

Die in Figur 6 dargestellte Laserschutzbrille (17) besitzt an ihren Ohrbügeln (18) jeweils eine Platte (19) mit Lüftungsschlitzen (20). Diese Lüftungsschlitze (20) ermöglichen eine gute Belüftung im Bereich zwischen Laserschutzbrille (17) und dem Gesicht des Brillenträgers und verhindern einen Hitzestau unter der Laserschutzbrille (17). Die Schutzfilter (22, 23) sind objektseitig konvex gewölbt, um durch divergente Reflexion die reflektierte Strahlungsdichte zu verringern. Das Fassungsteil (21) im Bereich der Schutzfilter (22, 23) weist deshalb zweckmäßigerweise auch eine entsprechende Wölbung auf. Im Fassungsteil (21) befindet sich zwischen den beiden Schutzfiltern (22, 23) ein waagrechter Schlitz (50), der zur Montage und Demontage der Schutzfilter (22, 23) notwendig ist. Nachdem die Montage der Schutzfilter (22, 23) in das Fassungsteil (21) der Laserschutzbrille (17) erfolgt ist, wird in den Schlitz (50) ein Abdeckungsteil (51) eingeführt. Dieses Abdeckungsteil (51) ist frontseitig verbreitert ausgeführt. Eine Schraube (49) geht vertikal durch den Nasensteg (52) und durch den Schlitz (50), wobei sie das eingesteckte Abdeckungsteil (51) fest im Schlitz (50) verankert. Die genaue Gestaltung des Ohrbügels (18) ist in den folgenden Figuren 7-9 dargestellt.

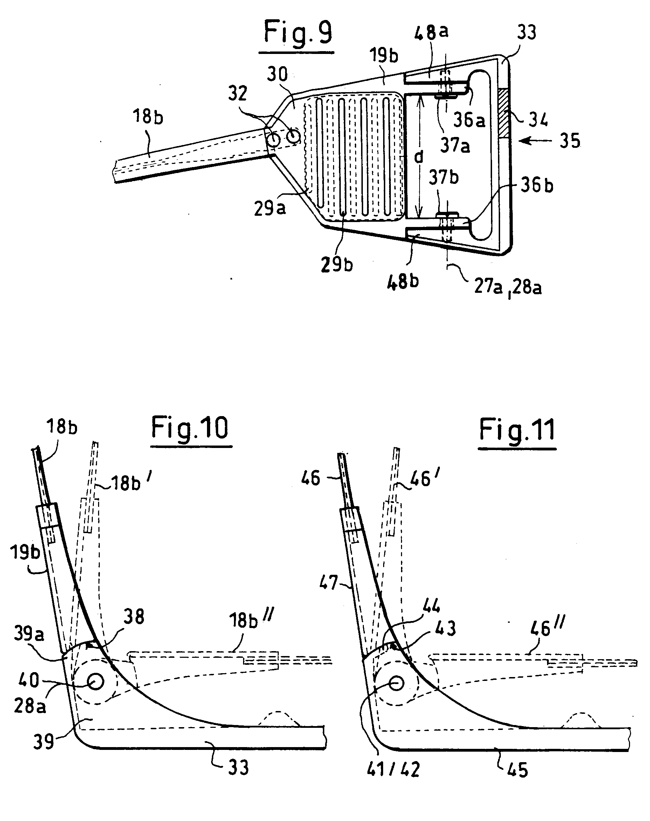

Das Fassungsteil (21) der Laserschutzbrille (17) mit den Schutzfiltern (22, 23) ist, bis auf den Bereich an den Ohrbügeln (17), wie in den Fig. 1-5 beschrieben gestaltet. Die unterschiedliche Befestigung der Ohrbügel (17) an der Schläfenplatte (24) des Fassungsteil (21) wird anhand der Figuren 10 und 11 näher erläutert.

In Figur 7 ist ein Ohrbügel (18a) dargestellt, welcher in der an ihm angebrachten Platte (19a) seitlich angebrachte Lüftungsschlitze (25) besitzt. Diese Lüftungsschlitze (25) haben eine Breite (b) und sind V-förmig ausgebildet mit einem Öffnungswinkel α = 60°. Durch die V-förmige Gestalt der Lüftungsschlitze (25) wird sichergestellt, daß keine Lichtstrahlung durch die Lüftungsschlitze (25) dringen kann. Die Lüftungsschlitze (25) stellen somit eine Lichtfalle dar. Sie sind dabei auf einen Bereich (c) der Platte (19a) begrenzt. Die Platte (19a) am Ohrbügel (18a) endet scharnierseitig mit einer gerundeten Fläche (26), deren Radiusmittelpunkt (27) mit dem Drehmittelpunkt (28) des Scharniers zusammenfällt.

Auch bei dem in Fig. 8 dargestellten Ohrbügel (18b) endet die am Ohrbügel (18a) angebrachte Platte (19a) scharnierseitig mit einer gerundeten Fläche (26), deren Radiusmittelpunkt (27a) mit dem Drehmittelpunkt (28a) des Scharniers zusammenfällt. Die am Ohrbügel (18a) angebrachte Platte (19a) besitzt sowohl äußere (29a) als auch innere (29b) Lüftungsschlitze (29). Die äußeren und inneren Lüftungsschlitze (29) befinden sich sowohl an der am Ohrbügel (18a) angebrachten Platte (19a) als auch an einer diskreten zweiten Platte (30) und sind durch einen Lüftungskanal (31) miteinander verbunden. Dabei sind die äußeren und inneren Lüftungsschlitze (29a, b) so zueinander angeordnet, daß ein Lichtstrahl nicht direkt von außen nach innen durch die Lüftungsschlitze (29) fallen kann. Die am Ohrbügel (18b) befestigte Platte (19b) besitzt am hinteren Ende zwei als Nieten (32) nutzbare Körper. Mit diesen Nieten (32) wird sowohl die zweite diskrete Platte (30) als auch der Ohrbügel (18b) an der Platte (19b) befestigt.

In Figur 9 ist ein Ohrbügel (18b) mit an ihm angebrachter Platte (19b) und Lüftungsschlitzen (29a, b) gemäß Fig. 8 in seitlicher Ansicht von innen mit durchgetrenntem Nasensteg (34) am Fassungsteil (33) der Laserschutzbrille (35) dargestellt. Deutlich ist hier zu sehen, daß die äußeren und inneren Lüftungsschlitze (29a, b) zueinander versetzt angeordnet sind.

Die Platte (19b) besitzt zwei Scharnierteile (36 bzw. 36a, b). Mit diesen Scharnierteilen (36) wird der Ohrbügel (18b) im oberen und unteren Bereich der Schläfenplatte (48a, b) drehbar befestigt. Dies erfolgt durch zwei Schrauben (37a, b), deren Gewinde nur in jeweils eine Gewindebohrung in der Schläfenplatte (48a, b) greift. Zwischen den beiden Scharnierteilen (36) besteht ein Abstand (d).

In Figur 10 ist der Ohrbügel (18b) mit an ihm angebrachter Platte (19b) aus Figur 8 und 9 in Ansicht von oben dargestellt. Die kreisrunde Gleitfläche (38) zwischen der Platte (19b) am Ohrbügel (18b) und der Schläfenplatte (39) des Fassungsteils (33) hat ihren Radiusmittelpunkt (40) im Drehmittelpunkt (28a) des Drehscharniers. Da die Schläfenplatte (39) in der Nähe der Gleitfläche (38) eine Verdickung (39a) besitzt, kann der Ohrbügel (18b) bis zur Stellung (18b') bewegt werden, ohne daß Strahlung im Bereich der Gleitfläche (38) eindringen kann. In der Stellung (18b'') befindet sich die Laserschutzbrille in ihrer Aufbewahrungsstellung.

Sollte es notwendig sein, daß man den Bügel (18b) über die in Fig. 10 dargestellte Stellung (18b') bewegen kann, ohne daß Lichtstrahlung an der Gleitfläche (38) ins Innere der Brillenfassung eindringen kann, so kann man die Verdickung (39a) auch noch vergrößern. Dies ist in Fig. 11 dargestellt.

In Fig. 11 kann man den Brillenbügel (46) noch über die Stellung (46') hinausbewegen, ohne daß Lichtstrahlung in das Innere der Laserschutzbrille (45) eindringen kann. Die Laserschutzbrille (45) besitzt an der Gleitfläche (43), deren Radiusmittelpunkt (41) auch hier im Drehmittelpunkt (42) des Drehscharniers ist, eine lippenartige Verlängerung (44). An dieser lippenartigen Verlängerung (44) kann die Platte (47) des Ohrbügels (46) entlanggleiten. Die Verlängerung (44) befindet sich dabei im Bereich (d) zwischen den beiden Scharnierteilen (36a, b in Fig. 9) an der Platte (47). In ihrer Aufbewahrungsstellung (46'') kann die Laserschutzbrille (45) in ihr Etui (hier nicht dargestellt) gesteckt werden.

Die dargestellten Fassungen haben den Vorteil, daß der die Schutzfilter aufnehmende Fassungsteil einstückig ausgebildet und demzufolge preiswert herstellbar ist. Dieser Fassungsteil schirmt den Bereich zwischen der Fassung und dem Gesicht des Trägers zuverlässig ab und bildet auch im Schläfenbereich Seitenblenden, d.h. er bietet Schutz vor schädlicher Strahlung die von vorn, von oben oder unten und von der Seite auftreffen kann. Dadurch, daß die Scharniere erst am ohrseitigen Ende der Seitenblenden angesetzt sind, besteht auch keine Gefahr, daß sich bei Bewegungen der Ohrbügel Schlitze im kritischen Bereich bilden.

Die Ohrbügel sind über Scharniere angesetzt, die im Inneren der Fassung angeordnet sind, wobei jeder Ohrbügel in eine Platte integriert ist, welche die Fortsetzung der zugeordneten Seitenblende des ersten Fassungsteils bildet. Dadurch ergibt sich von außen gesehen eine lange glatte Seitenblende, die nur durch eine Trennfuge unterbrochen ist. Durch eine spezielle Ausbildung der Trennfuge ist verhindert, daß sich bei geringen Bewegungen der Ohrbügel ein für Strahlung durchlässiger Schlitz bildet.

Durch eine Metalleinlage im Ohrbügel ist erreicht, daß sich die Ohrbügel biegen und damit der Kopfform des Brillenträgers anpassen lassen.

Der erste, einstückig ausgebildete Fassungsteil kann aus Leichtmetall, vorzugsweise aus Aluminium hergestellt werden. Als weiteres zweckmäßiges Material hat sich der unter dem Handelsnamen "Grilamid" erhältliche Kunststoff erwiesen, der für das erste Fassungsteil glasfaserverstärkt verwendet wird. Für die Brillenbügel findet vorteilhaft auch z.B. der Kunststoff "Grilamid" Verwendung, allerdings ohne Glasfaserverstärkung und es kann eine Metalleinlage eingesetzt sein.

Bei Verwendung des beschriebenen Kunststoffes läßt sich bei der Laserschutzbrille nach der Erfindung eine Schutzstufe besser als 7A für die Verwendung mit UV-Lasern erreichen. Eine ähnlich hohe Schutzstufe läßt sich auch für die Verwendung von CO₂-Lasern erreichen, wenn die Fassung aus Aluminium besteht.

In den Ohrbügeln sind Lüftungsschlitze vorhanden. Diese Lüftungsschlitze sind so gestaltet, daß durch sie keine Laserstrahlung in den Bereich zwischen Laserschutzbrille und Gesicht des Brillenträgers eindringen kann.

Das zur Schläfenplatte weisende Ohrbügelende sollte eine Rundung besitzen, deren Radiusmittelpunkt mit dem Drehpunkt des Scharniers übereinstimmt.

Desweiteren ist es vorteilhaft, die Befestigung der Ohrbügel an der Schläfenplatte der Schale mit den Schutzfiltern im oberen und unteren Bereich dieser Schläfenplatte vorzunehmen. Vorteilhafterweise besitzt die Schläfenplatte im Drehbereich der Ohrbügel eine lippenförmige Verlängerung.

Die in den Figuren dargestellte neue Laserschutzbrille kann beispielsweise aus Aluminium hergestellt werden. Es ist auch möglich die Fassung aus einem Kunststoff, vorzugsweise aus dem unter der Handelsbezeichnung "Grilamid" erhältlichen Kunststoff auszubilden. Dabei wird zweckmäßig das Fassungsteil aus glasfaserverstärktem Kunststoff gebildet, während die Ohrbügel und die integrierte Platte aus nichtglasfaserverstärktem Kunststoff gebildet werden. Weiterhin ist es vorteilhaft, die Ohrbügel in hier nicht dargestellter Weise mit einer Metalleinlage zu versehen, um sie in gewissem Umfang verformbar zu halten, so daß die Fassung dem Kopf des Brillenträgers genau angepaßt werden kann.

Die in den Fig. 7 und 8 dargestellte Formgestaltung der Lüftungsschlitze ist nur beispielhaft. Alle Formgestaltungen von Lüftungsschlitzen, welche einen direkten Durchgang von Lichtstrahlung verhindern, können verwendet werden.

## Patentansprüche

1. Schutzbrille (17) gegen augenschädliche Strahlung, bestehend aus einer Brillenfassung (21) mit Ohrbügeln (18, 18a, 18b), in welcher in der Brillenfassung Schutzfilter (22, 23) eingesetzt sind und bei welcher an den Ohrbügeln (18, 18a, 18b) der Schutzbrille (17) eine Platte (19, 19a, 19b) mit mehreren Lüftungsschlitzen (20, 25, 29a, 29b) angebracht ist, dadurch gekennzeichnet, daß die Lüftungsschlitze (20, 25, 29a, 29b) als Lichtfallen und somit lichtstrahlungsundurchlässig ausgebildet sind.

2. Schutzbrille gegen augenschädliche Strahlung nach Anspruch 1, dadurch gekennzeichnet, daß an der Schutzbrille (17) äußere und innere Lüftungsschlitze (20, 29a, 29b) angebracht sind, wobei diese Lüftungsschlitze (20, 29a, 29b) so zueinander angeordnet sind, daß ein Lichtstrahl nicht direkt von außen nach innen durch die Lüftungsschlitze (20, 29a, 29b) fallen kann.

3. Schutzbrille gegen augenschädliche Strahlung nach Anspruch 1, dadurch gekennzeichnet, daß die Lüftungsschlitze (20, 25) V-förmig ausgebildet sind und daß der Öffnungswinkel der V-förmigen Lüftungsschlitze (20, 25) so gewählt ist, daß keine Lichtstrahlung durch die Lüftungsschlitze (20, 25) fallen kann.

4. Schutzbrille gegen augenschädliche Strahlung nach Anspruch 2, dadurch gekennzeichnet, daß die äußeren und inneren Lüftungsschlitze (20, 29a, 29b) zueinander versetzt angeordnet sind.

5. Schutzbrille gegen augenschädliche Strahlung nach einem der Ansprüche 2 oder 4, dadurch gekennzeichnet, daß die äußeren Lüftungsschlitze (29a) auf einer äußeren Platte (19b) angebracht sind, daß die inneren Lüftungsschlitze (29b) auf einer inneren Platte (30) angebracht sind und daß zwischen den Platten (19b, 30) ein Lüftungskanal ausgebildet ist.

6. Schutzbrille gegen augenschädliche Strahlung nach Anspruch 5, dadurch gekennzeichnet, daß die Schutzfilter (22, 23) als Laserschutzfilter ausgebildet sind.

7. Schutzbrille gegen augenschädliche Strahlung nach einem der Ansprüche 2-6, dadurch gekennzeichnet, daß an der Schutzbrille (17) seitliche Platten (19) angebracht sind und daß die Lüftungsschlitze (20, 25, 29a, 29b) auf diesen Platten (19) ausgebildet sind.

## Claims

1. Safety spectacles (17) against harmful radiation for eyes consisting of a spectacle frame (21) with side arms (18, 18a, 18b) and serving to accommodate inserted protectiv filters (22, 23) wherein a plate (19, 19a, 19b) with more than one ventilation slits (20, 25, 29a, 29b) is connected to the side arms (18, 18a, 18b) of the safety spectacles (17), characterised by that the ventilation slits (20, 25, 29a, 29b) are constructed as light traps and therefor are impermeable to light radiation.

2. Safety spectacles against harmful radiation as claimed in claim 1, wherein outer and inner ventilation slits (20) are attached to the safety spectacles (17), mutually arranged in such a way that a light beam cannot fall directly from the outside to the inside through the ventilation slits (20).

3. Safety spectacles against harmful radiation as claimed in claim 1, wherein the ventilation slits (20, 25) are designed in the form of a V and the sice of the included angle of the V shape of the ventilation slits (20, 25) ensure that no light radiation can penetrate through the ventilation slits (20, 25).

4. Safety spectacles against harmful radiation as claimed in claim 2, wherein the outer and inner ventilation slits (20) are arranged mutually offset.

5. Safety spectacles against harmful radiation as claimed in claim 2 or 4, wherein the outer ventilation slits (29a) are attached to an outer plate (19b), wherein the inner ventilation slits (29b) are attached to an inner plate (30) and wherin a ventilation duct (31) is arranged between this plates (19b, 30).

6. Safety spectacle against harmful radiation as claimed in claim 5, wherein said protectiv filters (22, 23) are laser protectiv filters.

7. Safety spectacles against harmful radiation as claimed in one of claim 2-6, wherein lateral plates (19) are connected to the safety spectacles (17) and wherin the ventilation slots (20, 25, 29a, 29b) are attached on this plates (19).

## Revendications

1. Lunettes de protection (17) contre le rayonnement nuisible aux yeux, comprenant une monture (21) à branches (18, 18a, 18b), dans laquelle des filtres protecteurs (22, 23) sont insérés et sur laquelle une plaque (19, 19a, 19b) pourvue de plusieurs ouïes d'aération (20, 25, 29a, 29b) est fixée aux branches (18, 18a, 18b) des lunettes de protection (17), caractérisées en ce que les ouïes d'aération (20, 25, 29a, 29b) sont façonnées pour piéger la lumière et empêcher ainsi le passage d'un rayonnement lumineux.

2. Lunettes de protection contre le rayonnement nuisible aux yeux selon la revendication 1, caractérisées en ce que des ouïes d'aération (20) internes et externes sont aménagées sur les lunettes de protection (17) et disposées les unes par rapport aux autres de manière à ce que des rayons lumineux ne puissent traverser directement ces fentes (20) de l'extérieur vers l'intérieur.

3. Lunettes de protection contre le rayonnement nuisible aux yeux selon la revendication 1, caractérisées en ce que des ouïes d'aération (20, 25) présentent un profil en V et que l'angle d'ouverture de ces fentes en forme de V (20, 25) est choisi de façon à ce que des rayons lumineux ne puissent passer par ses ouïes d'aération (20, 25).

4. Lunettes de protection contre le rayonnement nuisible aux yeux selon la revendication 2, caractérisées en ce que des ouïes d'aération (20) internes et externes sont décalées les unes par rapport aux autres.

5. Lunettes de protection contre le rayonnement nuisible aux yeux selon les revendications 2 ou 4, caractérisées en ce que des ouïes d'aération (29a) externes sont aménagées sur une plaque externe (19b), que des ouïes d'aération (29b) internes sont disposées sur une plaque interne (30) et en ce qu'un canal d'aération est élaboré entre les plaques (19b, 30).

6. Lunettes de protection contre le rayonnement nuisible aux yeux selon la revendication 5, caractérisées en ce que les filtres protecteurs (22, 23) présentent la configuration de filtres de protection contre le rayonnement laser.

7. Lunettes de protection contre le rayonnement nuisible aux yeux selon les revendications numérotées de 2 à 6, caractérisées en ce que des plaques latérales (19) sont montées sur les lunettes de protection (17) et que des ouïes d'aération (20, 25, 29a, 29b) sont aménagées sur ces plaques (19).
